(19) Europäisches Patentamt
European Patent Office
Office européen des brevets

(11) Veröffentlichungsnummer: **0 082 812**
**B2**

# (12) NEUE EUROPÄISCHE PATENTSCHRIFT

(45) Veröffentlichungstag der neuen Patentschrift: **28.01.87**

(51) Int. Cl.⁴: **C 07 C 39/16**, C 07 C 39/17, C 07 C 43/164, C 07 C 149/10, C 08 K 5/13

(21) Anmeldenummer: **82810508.0**

(22) Anmeldetag: **29.11.82**

(54) **Phenole und ihre Verwendung als Stabilisatoren.**

(30) Priorität: **03.12.81 CH 7734/81**

(43) Veröffentlichungstag der Anmeldung: **29.06.83 Patentblatt 83/26**

(45) Bekanntmachung des Hinweises auf die Patenterteilung: **24.07.85 Patentblatt 85/30**

(45) Bekanntmachung des Hinweises auf die Entscheidung über den Einspruch: **28.01.87 Patentblatt 87/5**

(84) Benannte Vertragsstaaten: **BE DE FR GB IT NL**

(56) Entgegenhaltungen:
**EP - A - 0 003 338**
**GB - A - 1 229 574**
**US - A - 3 640 761**

**Journal of the Society of Chemical Industry Supplementary Issue, No. 1, 1950, S3-S7**
**Die Makromolekulare Chemie, Band IX, 1952, Seiten 1 bis 24**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Rosenberger, Siegfried, Im Gehracker 11, CH-4125 Riehen (CH)**

EP 0 082 812 B2

**Beschreibung**

Die Erfindung betrifft neue metafunktionelle, insbesondere an mindestens 2 aromatischen Kernen OH-Gruppen enthaltende Phenole, welche als Stabilisatoren für organisches Material Verwendung finden.

Es sind bereits einige mindestens an 2 aromatischen Kernen OH-Gruppen enthaltende Phenole bekannt, bei denen die aromatischen Kerne über Brückenradikale verbunden sind und welche die OH-Gruppe jeweils an den Kernen in o- oder p-Stellung zu dem C-Atom enthalten, über das die Verbrückung zum 2. aromatischen Kern erfolgt oder wo die OH-Substitution unbestimmt ist. Auch die Verwendung derartiger Phenole als Stabilisatoren ist bereits bekannt. Derartige Phenole sind beispielsweise in den folgenden Schutzrechtsschriften beschrieben: US Nr. 3 346 648, DE Nr. 2 138 839, JP Nr. 7 314 181.

Es sind auch bereits wenige mindestens an 2 aromatischen Kernen OH-Gruppen enthaltende Phenole bekannt, welche die OH-Gruppe an den Kernen in m-Stellung zu dem C-Atom enthalten, über das die Bindung zum 2. aromatischen Kern erfolgt. Auch die Verwendung solcher Phenole als Antioxidantien ist bekannt. Diesbezüglich ist auf DE Nr. 2 211 722 und auf eine Publikation von «H. Dudzikiewicz und J. Swoboda in Chem. Ber.» 98 (10) (1965) 3264-9 hinzuweisen.

Die erfindungsgemässen meta-funktionellen Phenole zeigen als Antioxidantien bessere Wirkungs- und Farbeigenschaften als die oben angeführten, bekannten Verbindungen.

In der Britischen Patentschrift GB Nr. 1 229 574 werden an 2 aromatischen Kernen OH-Gruppen enthaltende Phenole, welche am dritten, mittleren aromatischen Kern eine verätherte oder acylierte OH-Gruppe tragen, beschrieben. Auch solchen Verbindungen gegenüber zeigen die erfindungsgemässen Phenole eine überraschend bessere stabilisierende Wirkung.

Gegenstand der Erfindung sind Verbindungen der Formel I

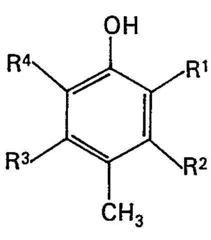

(I)

in der $R^1$ $C_1$–$C_4$-Alkyl, $R^4$ $C_1$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl ist, und $R^2$ und $R^3$ unabhängig voneinander einen der Reste der Formeln II, III oder IV darstellen,

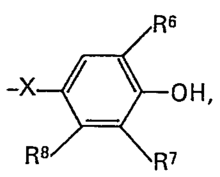

(II)

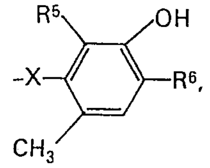

(III)

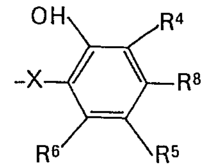

(IV)

wobei für $R^5$ die für $R^1$ angegebene Definition gilt und $R^5$ gleich oder verschieden von $R^1$ sein kann und für $R^6$ die für $R^4$ angegebene Definition gilt und $R^6$ gleich oder verschieden von $R^4$ sein kann, und X $-CH_2-$, $CH_2OCH_2-$ oder $-CH_2SCH_2-$ bedeutet, und $R^7$ $C_4$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl und $R^8$ Methyl oder Wasserstoff bedeutet, unter Ausschluss der Verbindung 3,5-Bis-(3-hydroxy-2,4,6-trimethylbenzyl)-2,4,6-trimethylphenol.

Bedeuten $R^1$, $R^4$, $R^5$, $R^6$ und $R^7$ $C_1$–$C_4$, $C_1$–$C_{12}$ bzw. $C_4$–$C_{12}$-Alkyl, so kann es sich z.B. um Methyl, Äthyl, n-Propyl, Isopropyl, n-Butyl, sec.-Butyl, tert.-Butyl, n-Pentyl, n-Hexyl, n-Octyl, 1,1,3,3-Tetramethylbutyl, n-Nonyl, Decyl oder Dodecyl handeln. $R^1$, $R^4$, $R^5$ und $R^6$ sind bevorzugt Methyl oder tert.-Butyl. Die bevorzugte Bedeutung von $R^7$ ist tert.-Butyl.

Bedeuten $R^4$, $R^6$ und $R^7$ $C_6$–$C_{10}$-Aryl, so kann es sich beispielsweise um Naphthyl und bevorzugt um Phenyl handeln.

Bedeuten $R^4$, $R^6$ und $R^7$ $C_7$–$C_{10}$-Aralkyl, so handelt es sich z.B. um Benzyl, 1-Phenyläthyl, α,α-Dimethylbenzyl oder um 2-Phenyläthyl.

$R^4$, $R^6$ und $R^7$ sind als $C_5$–$C_8$-Cycloalkyl z.B. Cyclopentyl, Cycloheptyl, Cyclooctyl und bevorzugt Cyclohexyl.

Als $C_7$–$C_{10}$-Alkaryl sind $R^4$, $R^6$ und $R^7$ beispielsweise Tolyl, 2,4-Dimethylphenyl, 2,6-Dimethylphenyl, 2,4-Diäthylphenyl, 2,6-Diäthylphenyl oder 4-tert.-Butylphenyl. Bevorzugt ist 2,4-Dimethylphenyl.

Bevorzugt werden Verbindungen der Formel I, worin $R^2$ und $R^3$ unabhängig voneinander einen Rest der Formeln II oder IV bedeutet und insbesondere solche, welche als $R^2$ und $R^3$ Reste der Formel II besitzen.

Bedeuten $R^2$ und/oder $R^3$ in Formel I Reste der Formel III, so sind in diesen die Substituenten $R^5$ und $R^6$ vorzugsweise $C_1$–$C_4$-Alkyl; die Reste $R^1$ und $R^4$ sind dann bevorzugt Methyl.

Die bevorzugte Bedeutung von X ist $-CH_2-$.

Eine Vorzugsform der Erfindung stellen solche Verbindungen der Formel I dar, wobei in Formel I $R^1$ und $R^5$ Methyl sind, und $R^4$ und $R^6$ unabhängig voneinander $C_1$–$C_4$-Alkyl sind, und $R^7$ tert.-Butyl ist, $R^8$ Wasserstoff bedeutet und $R^2$ und $R^3$ unabhängig voneinander Reste der Formeln II oder III bedeuten.

Beispiele der erfindungsgemässen Verbindungen sind folgende Substanzen.

Gemäss Formel I:

3,5-Di-(3',5'-di-t-butyl-4'-hydroxybenzyl)-2,4-dimethyl-6-t-butylphenol, 3,5-Di-(3',5'-di-t-butyl-4'-hydroxybenzyl)-2,4,6-trimethylphenol, 3,5-Di-(3'-methyl-5'-t-butyl-6'-hydroxybenzyl)-2,4,6-trimethylphenol, 3,5-Di-(2',6'-dimethyl-4'-t-butyl-3'-hydroxybenzyl)-2,4,6-trimethylphenol, 3,5-Di-(2',4'-di-t-butyl-6'-methyl-3'-hydroxybenzyl)-2,4,6-trimethylphenol.

Die erfindungsgemässen mehrkernigen Stabilisatoren der Formel I sind nach an sich bekannten Benzylierungsreaktionen an gehinderten Phenolen erhältlich, wobei ein Phenol der Formel V

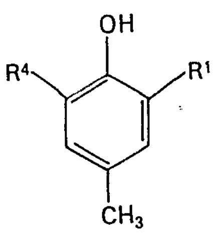

(V)

mit mindestens einer Benzylierungskomponente der Formeln VI, VII und/oder VIII

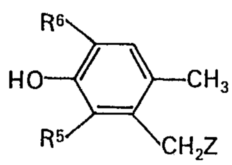

(VI)

(Z bedeutet Cl, Br, J, OH; vorzugsweise Cl)

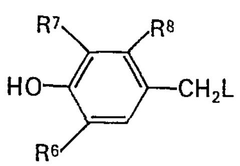

(VII)

(L bedeutet OH, $-OC_bH_{2b+1}$, $-N(C_bH_{2b+1})_2$; b = 1–4)

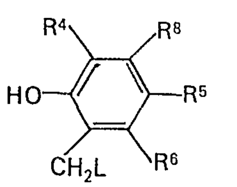

(VIII)

umgesetzt wird, wobei das molare Verhältnis der Verbindung der Formel V zu der Summe der Verbindungen der Formeln VI, VII und VIII ungefähr 1 : 2 ist.

Die Reaktionen werden gegebenenfalls in Gegenwart von Basen (z.B. t-Amine, Alkalien, Erdalkalien), ggf. aber auch in Gegenwart von sauren Katalysatoren, wie $H_2SO_4$, HCl, p-Toluolsulfonsäure $BF_3$-Ätherat, $ZnCl_2$, $AlCl_3$, und ggf. in Gegenwart eines inerten Lösungsmittels (z.B. Alkane, Aromaten, Äther, DMF, DMA) durchgeführt. Die Verbindungen der Formeln VI, VII und VIII können bei der Reaktion auch im Überschuss eingesetzt werden.

Als Ausgangsstoffe für die Herstellung der erfindungsgemässen Verbindungen werden bekannte Substanzen verwendet, deren Herstellung dem Fachmann ebenfalls bekannt ist.

Ein weiterer Gegenstand der Erfindung ist die Verwendung der in Anspruch 8 definierten Verbindungen der Formel IA als Stabilisatoren für organisches Material gegen dessen Schädigung durch Einwirkung von Sauerstoff, Wärme, Licht und energiereiche Strahlung, wie z.B. β- und γ-Strahlung.

Vorzugsformen dieser erfindungsgemässen Verwendung sind die Verwendung der Verbindungen als Stabilisatoren in organischen Polymeren, insbesondere in Pfropfpolymeren auf der Basis Acrylnitril, Butadien, Styrol (ABS) oder in schlagfestem Polystyrol.

Weitere Beispiele für organisches Material, welches mit den Verbindungen IA vorteilhaft stabilisiert werden kann, sind:

1. Polymere von Mono- und Diolefinen, beispielsweise Polyäthylen (das ggf. vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1 genannten Polymeren, z.B. Mischungen von Polypropylen mit Polyäthylen oder mit Polyisobutylen.

3. Copolymere von Mono- und Diolefinen untereinander oder mit anderen Vinylmonomeren, wie z.B. Äthylen-Propylen-Copolymere, Propylen-Buten-1-Copolymere, Propylen-Isobutylen-Copolymere, Äthylen-Buten-1-Copolymere, Propylen-Butadien-Copolymere, Isobutylen-Isopren-Copolymere, Äthylen-Alkylacrylat-Copolymere, Äthylen-Alkylmethacrylat-Copolymere, Äthylen-Vinylacetat-Copolymere oder Äthylen-Acrylsäure-Copolymere und deren Salze (Ionomere) sowie Terpolymere von Äthylen mit Propylen und einem Dien, wie Hexadien, Dicyclopentalien oder Äthylidennorbornen.

4. Polystyrol.

5. Copolymere von Styrol oder α-Methylstyrol mit Dienen oder Acrylderivaten, wie z.B. Styrol-Butadien, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Äthylen-Propylen-Dien-Terpolymeren; sowie Block-Copolymere des Styrols, wie z.B. Styrol-Butadien-Styrol, Styrol-Isopren-Styrol, Styrol-Äthylen/Butylen-Styrol oder Styrol-Äthylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Äthylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5 genannten Copolymeren, wie z.B. als sogenannte ABS, MBS oder AES-Polymere bekannt sind.

7. Halogenhaltige Polymere, wie z.B. Polychloropren, Chlorkautschuk, chloriertes oder chlorsulfoniertes Polyäthylen, Epichlorhydrinhomo- und copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und Derivaten ableiten, wie Polyacrylate und Polymethacrylate, Polyacrylamide und Polyacrylnitrile.

9. Copolymere der unter 8 genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere, oder Acrylnitril, Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymere von cyclischen Äthern, wie Polyalkylenglykole, Polyäthylenoxyd, Polypropylenoxyd oder deren Copolymere mit Bisglycidyläthern.

12. Polyacetale, wie Polyoxymethylen, sowie solche Polyoxymethylene, die Comonomere wie z.B. Äthylenoxyd enthalten.

13. Polyphenylenoxide und -sulfide sowie Mischungen von Polyphenylenoxiden mit Polystyrol.

14. Polyurethane, die sich von Polyäthern, Polyestern und Polybutadienen mit endständigen Hydroxylgruppen einerseits und aliphatischen oder aromatischen Polyisocyanaten andererseits ableiten sowie deren Vorprodukte (Polyisocyantate, Polyole, Präpolymere).

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid Poly-m-phenylenisophthalamid, sowie deren Copolymere mit Polyäthern wie z.B. mit Polyäthylenglykol Polypropylenglykol oder Polytetramethylenglykol.

16. Polyharnstoffe, Polyimide und Polyamidimide.

17. Polyester, die sich von Dicarbonsäuren und Diolen und/oder von Hydroxycarbonsäuren oder den entsprechenden Lactonen ableiten, wie Polyäthylenterephthalat, Polybutylenterephthalat, Poly-1,4-dimethylolcyclohexanterephthalat, Poly-2,2-bis-(4-hydroxyphenyl)propanterephthalat, Polyhydroxybenzoate, sowie Block-Polyätherester, die sich von Polyäthylen mit Hydroxyengruppen, Dialkoholen und Dicarbonsäuren ableiten.

18. Polycarbonate.

19. Polysulfone und Polyäthersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyl- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen sowie Vinylverbindungen als Vernetzungsmittel ableiten wie auch deren halogenhaltige, schwerbrennbare Modifikationen.

23. Vernetzbare-Acrylharze, die sich von substituierten Acrylsäureestern ableiten wie z.B. von Epoxyacrylaten, Üthanacrylaten oder Polyesteracrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycicyläthern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseäther, wie Methylcellulose.

27. Natürlich und synthetisch organische Stoffe, die reine monomere Verbindungen oder Mischungen von solchen darstellen, beispielsweise Mineralöle, tierische und pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Weichmacher für Kunststoffe oder als Spinpräparationen Anwendung finden, sowie deren wässerigen Emulsionen.

28. Wässerige Emulsionen natürlicher oder synthetischer Kautschuke wie z.B. Naturkautschuk-Latex oder Latices von carboxylierten Styrol-Butadien-Copolymeren.

Von besonderer Bedeutung ist die Stabilisierung von Styrolpolymeren und Elastomeren. Die erfindungsgemäss stabilisierten Polymeren weisen ein ausgezeichnetes Farbverhalten auf, und es besteht beste Verträglichkeit dieser Stabilisatoren mit den Polymeren.

Die Verbindungen der Formel IA sind auch besonders vorteilhaft als Stabilisatoren für Schmiermittel geeignet.

Die Stabilisatoren werden den Kunststoffen in einer Konzentration von 0,01 bis 5 Gew.%, berechnet auf das zu stabilisierende Material, zugesetzt. Vorzugsweise werden 0,1 bis 2,0, besonders bevorzugt 0,2 bis 0,6 Gew.% der Verbindungen, berechnet auf das stabilisierende Material, in dieses eingearbeitet.

Die Einarbeitung kann nach der Polymerisation erfolgen, beispielsweise durch Einmischen der

Verbindungen und ggf. weiterer Additive in die Schmelze nach den in der Technik üblichen Methoden, vor oder während der Formgebung, oder auch durch Aufbringen der gelösten oder dispergierten Verbindungen auf das Polymere, ggf. unter nachträglichem Verdunsten des Lösungsmittels.

Die Erfindung betrifft daher auch die durch Zusatz einer Verbindung der Formel IA stabilisierten organischen Polymeren, die ggf. noch andere bekannte und übliche Zusätze enthalten können. Die so stabilisierten Kunststoffe können in verschiedenster Form angewendet werden, z.B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

1. Antioxidantien

1.1. Alkylierte Monophenole

2,6-Di-tert.-butyl-4-methylphenol
2-Tert.-butyl-4,6-dimethylphenol
2,6-Di-tert.-butyl-4-äthylphenol
2,6-Di-tert.-butyl-4-n-butylphenol
2,6-Di-tert.-butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.-butyl-4-methoxymethylphenol
2,6-Diphenyl-4-octadecyloxyphenol

1.2. Alkylierte Hydrochinone

2,6-Di-tert.-butyl-4-methoxyphenol
2,5-Di-tert.-butylhydrochinon
2,5-Di-tert.-amylhydrochinon

1.3. Hydroxylierte Thiodiphenyläther

2,2′-Thio-bis-(6-tert.-butyl-4-methylphenol)
2,2′-Thio-bis-(4-octylphenol)
4,4′-Thio-bis-(6-tert.-butyl-3-methylphenol)
4,4′-Thio-bis-(6-tert.-butyl-2-methylphenol)

1.4. Alkyliden-Bisphenole

2,2′-Methylen-bis-(6-tert.-butyl-4-methylphenol)
2,2′-Methylen-bis-(6-tert.-butyl-4-äthylphenol)
2,2′-Methylen-bis-[4-methyl-6-(α-methylcyclohexyl)phenol]
2,2′-Methylen-bis-(4-methyl-6-cyclohexylphenol)
2,2′-Methylen-bis-(6-nonyl-4-methylphenol)
2,2′-Methylen-bis-(4,6-di-tert.-butylphenol)
2,2′-Äthyliden-bis-(4,6-di-tert.-butylphenol)
2,2′-Äthyliden-bis-(6-tert.-butyl-4-isobutylphenol)
4,4′-Methylen-bis-(2,6-di-tert.-butylphenol)
4,4′-Methylen-bis-(6-tert.-butyl-2-methylphenol)
1,1-Bis-(5-tert.-butyl-4-hydroxy-2-methylphenyl)butan
2,6-Di-(3-tert.-butyl-5-methyl-2-hydroxybenzyl)-4-methylphenol
1,1,3-Tris-(5-tert.-butyl-4-hydroxy-2-methylphenyl)butan
1,1-Bis-(5-tert.-butyl-4-hydroxy-2-methylphenyl)-3-n-dodecylmercaptobutan
Äthylenglykol-bis-[3,3-bis-(3′-tert.-butyl-4′-hydroxyphenyl)butyrat]
Di-(3-tert.-butyl-4-hydroxy-5-methylphenyl)-dicyclopentadien
Di-[2-(3′-tert.-butyl-2′-hydroxy-5′-methylbenzyl)-6-tert.-butyl-4-methylphenyl]-terephthalat

1.5. Benzylverbindungen

1,3,5-Tri-(3,5-di-tert.-butyl-4-hydroxybenzyl)-2,4,6-trimethylbenzol
Di-(3,5-di-tert.-butyl-4-hydroxybenzyl)sulfid
3,5-di-tert.-butyl-4-hydroxybenzylmercaptoessigsäure-isooctylester
Bis-(4-tert.-butyl-3-hydroxy-2,6-dimethylbenzyl)dithiolterephthalat
1,3,5-Tris-(3,5-di-tert.-butyl-4-hydroxybenzyl)isocyanurat
1,3,5-Tris-(4-tert.-butyl-3-hydroxy-2,6-dimethylbenzyl)isocyanurat
3,5-Di-tert.-butyl-4-hydroxybenzylphosphonsäure-dioctadecylester
3,5-Di-tert.-butyl-4-hydroxybenzylphosphonsäure-monoäthylester, Calciumsalz.

1.6. Acylaminophenole

4-Hydroxylaurinsäureanilid
4-Hydroxystearinsäureanilid
2,4-Bis-octylmercapto-6-(3,5-di-tert.-butyl-4-hydroxyanilino)-S-triazin

1.7. Ester der β-(3,5-Di-tert.-butyl-4-hydroxyphenyl)propionsäure
mit ein- oder mehrwertigen Alkoholen, wie z.B. mit

| | |
|---|---|
| Methanol | Diäthylenglycol |
| Octadecanol | Triäthylenglycol |
| 1,6-Hexandiol | Pentaerythrit |
| Neopentylglycol | Tris-hydroxyäthylisocyanurat |
| Thiodiäthylenglycol | Di-hydroxyäthyloxalsäurediamid |

1.8. Ester der β-(5-tert.-butyl-4-hydroxy-3-methylphenyl)propionsäure
mit ein- oder mehrwertigen Alkoholen, wie z.B. mit

| | |
|---|---|
| Methanol | Diäthylenglycol |
| Octadecanol | Triäthylenglycol |
| 1,6-Hexandiol | Pentaerythrit |
| Neopentylglykol | Tris-hydroxyäthylisocyanurat |
| Thiodiäthylenglykol | Di-hydroxyäthyloxalsäurediamid |

1.9. Amide der β-(3,5-Di-tert.-butyl-4-hydroxyphenyl)propionsäure
wie z.B.

N,N′-Di-3,5-di-tert.-butyl-4-hydroxyphenylpropionyl)hexamethylendiamin
N,N′-Di-(3,5-di-tert.-butyl-4-hydroxyphenylpropionyl)trimethlendiamin
N,N′-Di-(3,5-di-tert.-butyl-4-hydroxyphenylpropionyl)hydrazin

2. UV-Absorber und Lichtschutzmittel

2.1. 2-(2'-Hydroxyphenyl)benztriazole, wie z.B. das 5'-Methyl-, 3',5'-Di-tert.-butyl-, 5'-Tert.-butyl-, 5'-(1,1,3,3-Tetramethylbutyl)-, 5-Chlor-3',5'-di-tert.-butyl-, 5-Chlor-3'-tert.-butyl-5'-methyl-, 3'-sec.-Butyl-5'-tert.-butyl-, 4'-Octoxy-3',5'-Ditert.-amyl-.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxyderivat.

2.3. Ester von ggf. substituierten Benzoesäuren, wie z.B. 4-Tert.-butyl-phenylsalicylat, Phenylsalicylat, Octylphenylsalicylat, Dibenzoylresorcin, Bis-(4-tert.-butylbenzol)resorcin, Benzoylresorcin, 3,5-Di-tert.-butyl-4-hydroxybenzoesäure-2,4-di-tert.-butylphenylester.

2.4. Acrylate, wie z.B. α-Cyan-β,β-diphenylacrylsäureäthylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäuremethylester.
N-(β-Carbomethoxy-β-cyanovinyl)-2-methylindolin

2.5. Nickelverbindungen, z.B. Nickelkomplexe des 2,2'-Thio-bis-[4-(1,1,3,3-tetramethylbutyl)phenols], wie der 1:1- oder der 1:2-Komplex, ggf. mit zusätzlichen Liganden wie n-Butylamin, Triäthanolamin oder N-Cyclohexyldiäthanolamin, Nickeldibutyldithiocarbamat, Nickelsalze von 4-Hydroxy-3,5-di-tert.-butylbenzylphosphonsäure Monoalkylestern wie vom Methyl- oder Äthylester, Nickelkomplexe von Ketoximen wie von 2-Hydroxy-4-methylphenylundecylketonoxim, Nickelkomplexe des 1-Phenyl-4-lauroyl-5-hydroxypyrazols, ggf. mit zusätzlichen Liganden.

2.6. Sterisch gehinderte Amine, wie z.B. Bis-(2,2,6,6-tetramethylpiperidyl)sebacat
Bis-(1,2,2,6,6-pentamethylpiperidyl)sebacat
n-Butyl-3,5-di-tert.-butyl-4-hydroxybenzylmalonsäure-bis-(1,2,2,6,6-pentamethylpiperidyl) ester Kondensationsprodukt aus 1-Hydroxyäthyl-2,2,6,6-Tetramethyl-4-hydroxypiperidin und Bernsteinsäure Kondensationsprodukt aus N,N'-(2,2,6,6-Tetramethylpiperidyl)-hexamethylendiamin und 4-tert.-octylamino-2,6-dichlor-1,3,5-s-triazin.
Tris-(2,2,6,6-tetramethylpiperidyl)nitrilotriacetat.

2.7. Oxalsäurediamide, wie z.B. 4,4'-Di-octyloxyoxanilid, 2,2'-Di-octyloxy-5,5'-di-tert.-butyloxanilid, 2,2'-Di-dodecyloxy-5,5'-di-tert.-butyloxanilid, 2-Äthoxy-2'-äthyloxanilid, N,N'-Bis-(3-dimethylaminopropyl)oxalamid, 2-Äthoxy-5-tert.-butyl-2'-äthyloxanilid und dessen Gemisch mit 2-Äthoxy-2'-äthyl-5,4'-di-tert.-butyloxanilid, Gemische von ortho- und para-Methoxy- sowie von o- und p-Äthoxy-disubstituierte Oxaniliden.

3. Metalldesaktivatoren, wie z.B. N,N'-Diphenyloxalsäurediamid, N-Salicylal-N'-salicyloylhyrazin, N,N'-Bis-salicyloylhydrazin, N,N'-Bis-(3,5-di-tert.-butyl-4-hydroxyphenylpropionyl)hydrazin, 3-Salicyloylamino-1,2,4-triazol, Bis-benzylidenoxalsäuredihydrazid.

4. Phosphite und Phosphonite, wie z.B. Triphenylphosphit, Diphenylalkylphosphite, Phenyldialkylphosphite, Tri-(nonylphenyl)phosphit, Trilaurylphosphit, Trioctadecylphosphit, Distearylpentaerythritdiphosphit, Tris-(2,4-di-tert.-butylphenyl)phosphit, Diisodecyl-pentaerythritdiphosphit, Di-(2,4-tert.-butylphenyl)pentaerythritdiphosphit, Tristearyl-sorbit-triphosphit, Tetrakis-(2,4-di-tert.-butylphenyl)-4,4'-biphenylendiphos phonit.

5. Peroxidzerstörende Verbindungen, wie z.B. Ester der β-Thio-di-propionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit- tetrakis-(β-dodecylmercapto)propionat.

6. Polyamidstabilisatoren, wie z.B. Kupfersalze in Kombination mit Jodiden und/oder Phosphorverbindungen und Salz des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.-Butylbenzoesäure, Adipinsäure, Diphenylessigsäure.

9. Füllstoffe und Verstärkungsmittel, wie z.B. Calciumcarbonate, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Beispiel 1

3,5-Di(3',5'-di-t-butyl-4-hydroxybenzyl)-2,4-dimethyl-6-t-butylphenol

21,5 g 2,4-Dimethyl-6-t-butylphenol werden in 60 ml Methanol gelöst und mit 11 g 80%iger Schwefelsäure versetzt. Dann werden bei ca. 70°C (Rückfluss) unter Rühren und Stickstoff innerhalb 6 h 60 g 4-Methoxymethyl-2,6-di-t-butylphenol zugegeben. Das Reaktionsgemisch wird weitere 6 h bei 70°C gehalten. Nach dem Abkühlen kristallisiert das Reaktionsprodukt in farblosen Kristallen aus. Es wird abgenutscht und durch Digerieren mit Hexan, Abnutschen, Digerieren mit NaOH-haltigem Wasser und abermaligem Abnutschen gereinigt. Smp. 140°C (aus Methanol).

Beispiel 2

Verwendet man bei analoger Arbeitsweise wie in Beispiel 1 statt 2,4-Dimethyl-6-butylphenol das 2,4,6-Trimethylphenol in aliquoter Menge, so erhält man das 3,5-Di-(3′,5′-di-t-butyl-4-hydroxybenzyl)-2,4,6-trimethylphenol vom Smp. 204°C.

Beispiel 3

100 Gewichtsteile unstabilisiertes ABS-Pulver werden mit dem in den Tabellen I und II angegebenen Stabilisator gemischt.

Das erhaltene Gemisch wird auf einem Zweiwalzenstuhl während 5 min bei maximal 170°C compoundiert und die Felle anschliessend abgezogen. Die Rohfelle werden auf einer hydraulischen Laborpresse bei 180°C während 6 min zu 1 mm dicken Platten verpresst, aus welchen Prüflinge der Dimension 50 × 20 mm gestanzt werden.

Die Prüfung der Wirksamkeit des den Prüflingen zugesetzten Stabilisators wird durch Hitzealterung in einem Umluftofen bei 180°C vorgenommen. Als Kriterium für die während der Alterung eingetretene Schädigung (Oxidation) dient das Infrarot-Absorptionsspektrum der Oberfläche, welches durch Reflexions-Spektroskopie (ATR) erhalten wird. Insbesondere wird die Zunahme der Carbonylextinktion (1720 $cm^{-1}$) als Funktion der Zeit verfolgt und mit einer konstant bleibenden Absorptionsbande (1455 $cm^{-1}$) verglichen. Dabei gilt als Mass für den Abbau:

$$V = \frac{\text{Opt. Dichte bei 1720 cm}^{-1}\ (> C = 0)}{\text{Opt. Dichte bei 1455 cm}^{-1}\ (> CH_2)}$$

Als willkürlicher Endpunkt wird die Zeit gewählt, nach welcher V den Wert 0,1 erreicht ($t_{0,1}$).

Tabelle 1 Prüfung in ABS ohne Synergist

| Stabilisator nach Herst. Beisp. Nr. 0,25 Gew.% | Ofenalterung 180°C | | | | | | |
|---|---|---|---|---|---|---|---|
| | | Y.I. ASTM D 1925 nach Min. Ofenalterung | | | | | |
| | $t_{0,1}$ 0 | 30 | 60 | 90 | 120 | 150 | |
| 1 | 70′ | 13,5 | 26,4 | 30 | 55,1 | 69,1 | — |
| 2 | 35′ | 19,7 | 33 | 48 | 69 | — | — |
| ohne Stabilisator | 7′ | 17 | 53 | 73 | 83 | — | — |

Tabelle 2 Prüfung in ABS mit dem Synergist DLTDP (Dilaurylthiodipropionat)

| Stabilisator nach Herst. Beisp. Nr. + DLTP 0,25 Gew.% Stab. 0,5 Gew.% DLTDP | Ofenalterung 180°C | | | | | | |
|---|---|---|---|---|---|---|---|
| | | Y.I. ASTM nach Min. Ofenalterung | | | | | |
| | $t_{0,1}$ | 0 | 30 | 60 | 90 | 120 | 150 |
| 1 | 107′ | 15 | 21 | 27,3 | 29,8 | 59,5 | — |
| 2 | 83′ | 18,2 | 31 | 37 | 51 | — | — |
| ohne Stabilisator | 7′ | 17 | 53 | 73 | 83 | — | — |

Beispiel 4

Ein schlagfestes Polystyrol mit 8 Gew.% Polybutadien-Gehalt (high-cis) und mit 0,035 Gew.% 2,6-Di-tert.-butyl-p-cresol als Grundstabilisator, 0,05 Gew.% Zinkstearat als Gleitmittel sowie 0,1 Gew.% eines der erfindungsgemässen Antioxidantien (in den nachfolgenden Tabellen III und IV jeweils mit der Nummer des entsprechenden Herstellungsbeispiels gekennzeichnet), wird zweimal bei 220°C extrudiert und das erhaltene Granulat bei 185°C in 3 min zu 2 mm dicken Prüfplättchen verpresst.

Die Prüflinge werden einer Ofenalterung im Umluftofen unterzogen und

a) Der Yellowness Index gemäss ASTM D 1925 bei 80°C (Messung der Prüflinge nach 0, 250, 500, 750 und 1000 h) und bei 160°C (Messung der Prüflinge nach 0, 60, 90, 120 und 180 min) bestimmt. Die Resultate sind in Tabelle III wiedergegeben.

b) die Schlagzähigkeit (SZ) in Kp. $cm/cm^2$ nach Alterung bei 160°C (Messung der Prüflinge nach 30, 60, 120, 150, 180, 240, 300, 360, 420, 480 min) bestimmt. Letztere Resultate sind in Tabelle IV zusammengestellt.

Tabelle 3

| Stabilisator nach Herst. Beisp. Nr. | Y.I. bei 80° C nach Stunden | | | | | Y.I. bei 160°C nach Minuten | | | | |
|---|---|---|---|---|---|---|---|---|---|---|
| | 0 | 250 | 500 | 750 | 1000 | 0 | 60 | 90 | 120 | 180 |
| ohne Stabilisator | 0,5 | 18 | 24 | 36 | 48 | 0 | 17 | 37 | 47 | 73 |
| 1 | 0,8 | 15 | 22 | 29 | 37 | 1 | 17 | 22 | 29 | 36 |

Tabelle 4

| Stabilisator nach Herst. Beisp. Nr. | SZ nach Alterung bei 160°C nach Minuten | | | | | | | | | |
|---|---|---|---|---|---|---|---|---|---|---|
| | 30 | 60 | 120 | 150 | 180 | 240 | 300 | 360 | 420 | 480 |
| ohne Stabilisator | 10,4 | 10,4 | — | — | — | — | — | — | — | — |
| 1 | X | X | X | X | X | X | X | X | 7,9 | — |

X bedeutet: Prüfling nicht gebrochen.

**Patentansprüche**

1. Verbindungen der Formel (I)

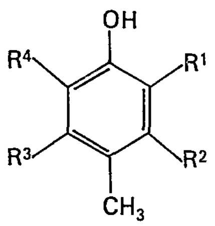

(I)

in der $R^1$ $C_1$–$C_4$-Alkyl, $R^4$ $C_1$–$C_{12}$-Alkyl, $C_5$–$C_8$-Cycloalkyl, $C_6$–$C_{10}$-Aryl, $C_7$–$C_{10}$-Aralkyl oder$C_7$–$C_{10}$-Alkaryl ist und $R^2$ und $R^3$ unabhängig voneinander einen der Reste der Formeln (II), (III) oder (IV) darstellen,

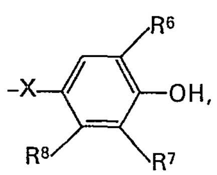

(II)

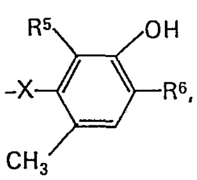

(III)

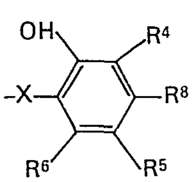

(IV)

wobei für $R^5$ die für $R^1$ angegebene Definition gilt und $R^5$ gleich oder verschieden von $R^1$ sein kann und für $R^6$ die für $R^4$ angegebene Definition gilt und $R^6$ gleich oder verschieden von $R^4$ sein kann, und X-$CH_2$–, -$CH_2OCH_2$– oder -$CH_2SCH_2$– bedeutet, und $R^7$ $C_4$–$C_{12}$-Alkyl, $C_5$–$C_8$-Cycloalkyl,$C_6$–$C_{10}$-Aryl, $C_7$–$C_{10}$-Aralkyl oder $C_7$–$C_{10}$-Alkaryl und $R^8$ Methyl oder Wasserstoff ist, unter Ausschluss der Verbindung 3,5-Bis-(3-hydroxy-2,4,6-trimethylbenzyl)-2,4,6-trimethylphenol.

2. Verbindungen gemäss Anspruch 1 der Formel (I), worin $R^2$ und $R^3$ unabhängig voneinander Reste der Formeln (II) oder (IV) bedeuten.

3. Verbindungen gemäss Anspruch 1 der Formel (I), worin $R^2$ und $R^3$ Reste der Formel (II) sind.

4. Verbindungen gemäss Anspruch 1 der Formel (I), worin $R^1$ und $R^4$ Methyl bedeuten und $R^2$ und $R^3$ Reste der Formel (III) sind, worin $R^5$ und $R^6$ $C_1$–$C_4$-Alkyl bedeuten.

5. Verbindungen gemäss Anspruch 1 der Formel (I), worin $R^2$ und $R^3$ Reste der Formeln (II), (III) oder (IV) sind, worin X-$CH_2$– bedeutet.

6. Verbindungen gemäss Anspruch 1 der Formel (I), worin $R^1$ und $R^5$ Methyl sind, und $R^4$ und $R^6$ unabhängig voneinander $C_1$–$C_4$-Alkyl sind, und $R^7$ t-Butyl ist, $R^8$ Wasserstoff bedeutet und $R^2$ und $R^3$ unabhängig voneinander Reste der Formel (II) oder (III) bedeuten.

7. 3,5-Di(3′,5′-di-t-butyl-4-hydroxybenzyl) 2,4,6-trimethylphenol gemäss Anspruch 1.

8. Verwendung der Verbindungen der Formel (IA)

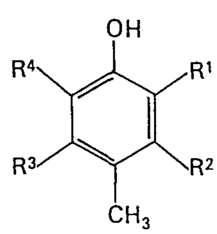

(IA)

in der $R^1$ $C_1$–$C_4$-Alkyl, $R^4$ $C_1$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl ist, und $R^1$ und $R^3$ unabhängig voneinander einen der Reste der Formeln (II), (III) oder (IV) darstellen,

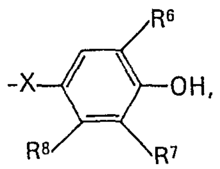

(II)

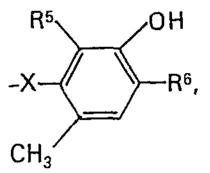

(III)

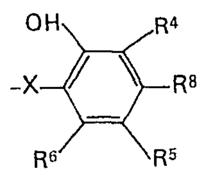

(IV)

wobei für $R^5$ die für $R^1$ angegebene Definition gilt und $R^5$ gleich oder verschieden von $R^1$ sein kann und für $R^6$ die für $R^4$ angegebene Definition gilt und $R^6$ gleich oder verschieden und $R^4$ sein kann, und X-$CH_2$–, -$CH_2OCH_2$– oder -$CH_2SCH_2$– bedeutet, und $R^7$ $C_4$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl und $R^8$ Methyl oder Wasserstoff ist, als Stabilisatoren für organisches Material.

9. Mit Verbindungen der in Anspruch 8 definierten Formel stabilisierte organische Polymere.

10. Stabilisierte Polymere gemäss Anspruch 9, dadurch gekennzeichnet, dass das Polymere ein pfropfpolymer auf der Basis Acrylnitril, Butadien, Styrol (ABS) oder ein schlagfestes Polystyrol ist.

11. Mit Verbindungen der in Anspruch 8 definierten Formel stabilisierte Schmiermittel.

**Claims**

1. A compound of the formula I

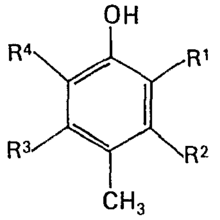

(I)

wherein $R^1$ is $C_1$–$C_4$ alkyl, $R^4$ is $C_1$–$C_{12}$ alkyl, $C_5$–$C_8$ cycloalkyl, $C_6$–$C_{10}$ aryl, $C_7$–$C_{10}$ aralkyl or $C_7$–$C_{10}$ alkaryl, and each of $R^2$ and $R^3$ independently of the other is one of the radicals of the formulae II, III or IV

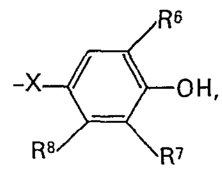

(II)

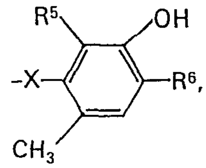

(III)

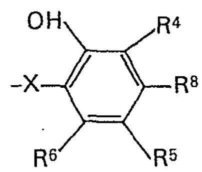

(IV)

wherein $R^5$ and $R^6$ are as defined above for $R^1$ and $R^4$ and both radicals may be identical to or different from $R^1$and $R^4$, and X is -$CH_2$–, -$CH_2OCH_2$– or -$CH_2SCH_2$–, $R^7$ is $C_4$–$C_{12}$ alkyl, $C_5$–$C_8$ cycloalkyl, $C_6$–$C_{10}$ aryl, $C_7$–$C_{10}$ aralkyl or $C_7$–$C_{10}$ alkaryl and $R^8$ is methyl or hydrogen, with the exclusion of the compound 3,5-bis-(3-hydroxy-2,4,6-trimethylbenzyl)-2,4,6-trimethylphenol.

2. A compound according to claim 1 of the formula I, wherein each of $R^2$ and $R^3$ independently of the other is a radical of the formula II or IV.

3. A compound according to claim 1 of the formula I, wherein each of $R^2$ and $R^3$ is a radical of the formula II.

4. A compound according to claim 1 of the formula I, wherein each of $R^1$ and $R^4$ is methyl and each of $R^2$ and $R^3$ is a radical of the formula III, wherein each of $R^5$ and $R^6$ is $C_1$–$C_4$ alkyl.

5. A compound according to claim 1 of the formula I, wherein each of $R^2$ and $R^3$ independently of the other is a radical of the formulae II, III or IV, wherein X is -$CH_2$–.

6. A compound according to claim 1 of the formula I, wherein each of $R^1$ and $R^5$ is methyl, each of $R^4$ and $R^6$ independently of the other is $C_1$–$C_4$ alkyl, $R^7$ is tert-butyl, $R^8$ is hydrogen and each of $R^2$ and $R^3$ independently of the other is a radical of the formula II or III.

7. 3,5-Di-(3′,5′-di-tert-butyl-4-hydroxybenzyl)-2,4,6-trimethylphenol according to claim 1.

8. A method of stabilising organic material which comprises the use of a compound of the formula IA

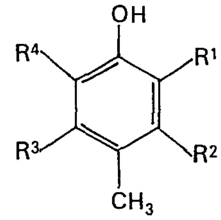

(IA)

wherein $R^1$ is $C_1$–$C_4$ alkyl, $R^4$ is $C_1$–$C_{12}$ alkyl, $C_5$–$C_8$ cycloalkyl, $C_6$–$C_{10}$ aryl, $C_7$–$C_{10}$ aralkyl or $C_7$–$C_{10}$ alkaryl, and each of $R^2$ and $R^3$ independently of the other is one of the radicals of the formulae II, III or IV

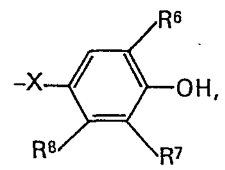

(II)

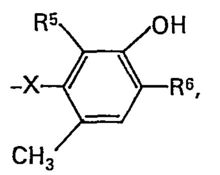

(III)

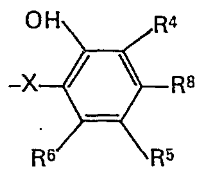

(IV)

wherein $R^5$ and $R^6$ are as defined above for $R^1$ and $R^4$ and both radicals may be identical to or different from $R^1$ and $R^4$, and X is $-CH_2-$, $-CH_2OCH_2-$ or $-CH_2SCH_2-$, $R^7$ is $C_4$–$C_{12}$ alkyl, $C_5$–$C_8$ cycloalkyl, $C_6$–$C_{10}$ aryl, $C_7$–$C_{10}$ aralkyl or $C_7$–$C_{10}$ alkaryl and $R^8$ is methyl or hydrogen.

9. An organic polymer stabilised with a compound of the formula as defined in claim 8.

10. A stabilised polymer according to claim 9, which polymer is a graft polymer based on acrylonitrile, butadiene, styrene (ABS) or an impact-resistant polystyrene.

11. A lubricant stabilised with a compound of the formula as defined in claim 8.

## Revendications

1. Composés répondant à la formule I:

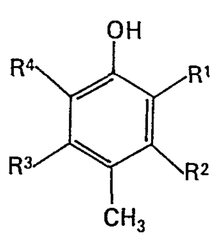

(I)

dans laquelle $R^1$ représente un alkyle en $C_1$–$C_4$, $R^4$ un alkyle en $C_1$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$, et $R^2$ et $R^3$ représentent chacun, indépendamment l'un de l'autre, un radical répondant à l'une des formules II, III et IV:

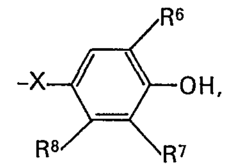

(II)

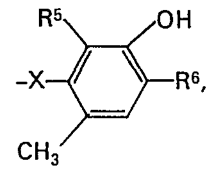

(III)

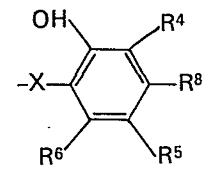

(IV)

dans lesquelles $R^5$ répond à la définition donnée ci-dessus pour $R^1$ et peut être identique ou différent de $R^1$, $R^6$ répond à la définition donnée ci-dessus pour $R^4$ et peut être identique ou différent de $R^4$, X représente un radical $-CH_2-$, $-CH_2OCH_2-$ ou $-CH_2SCH_2-$, $R^7$ représente un alkyle en $C_4$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$, et $R^8$ représente un méthyle ou l'hydrogène, sauf le bis-(hydroxy-3 triméthyl-2,4,6 benzyl)-3,5 triméthyl-2,4,6 phénol.

2. Composés de formule I selon la revendication 1, dans lesquels $R^2$ et $R^3$ représentent chacun, indépendamment l'un de l'autre, un radical répondant à l'une des formules II et IV.

3. Composés de formule I selon la revendication 1, dans lesquels $R^2$ et $R^3$ sont des radicaux de formule II.

4. Composés de formule I selon la revendication 1, dans lesquels $R^1$ et $R^4$ représentent chacun un radical méthyle tandis que $R^2$ et $R^3$ représentent des radicaux de formule III dans lesquels $R^5$ et $R^6$ représentent chacun un alkyle en $C_1$–$C_4$.

5. Composés de formule I selon la revendication 1, dans lesquels $R^2$ et $R^3$ représentent chacun un radical répondant à l'une des formules II, III et IV dans lesquelles X représente un radical $-CH_2-$.

6. Composés de formule I selon la revendication 1, dans lesquels $R^1$ et $R^5$ représentent chacun un méthyle, $R^4$ et $R^6$ représentent chacun, indépendamment l'un de l'autre, un alkyle en $C_1$–$C_4$, $R^7$ représente un tert-butyle, $R^8$ représente l'hydrogène, et $R^2$ et $R^3$ représentent chacun, indépendamment l'un de l'autre, un radical répondant à l'une des formules II et III.

7. Composé selon la revendication 1, en l'espèce le bis-(dotert-buty-3,5 hydroxy-4 benzyl)-3,5 triméthyl-2,4,6 phénol.

8. Application des composés répondant à la formule IA

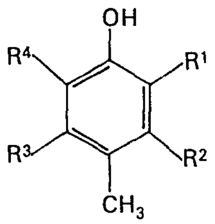

(IA)

dans laquelle $R^1$ représente un alkyle en $C_1$–$C_4$, $R^4$ un alkyle en $C_1$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$, et $R^2$ et $R^3$ représentent chacun, indépendamment l'un de l'autre, un radical répondant à l'une des formules II, III et IV:

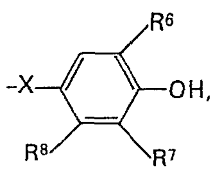

(II)

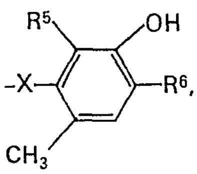

(III)

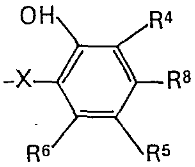

(IV)

dans lesquelles $R^5$ répond à la définition donnée ci-dessus pour $R^1$ et peut être identique ou différent de $R^1$, $R^6$ répond à la dénifition donnée ci-dessus pour $R^4$ et peut être identique ou différent de $R^4$, X représente un radical $-CH_2-$, $-CH_2OCH_2-$ ou $-CH_2SCH_2-$, $R^7$ représente un alkyle en $C_4$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$, et $R^8$ représente un méthyle ou l'hydrogène, en tant que stabilisants pour matières organiques.

9. Polymères organiques qui ont été stabilisés au moyen de composés répondant à la formule définie à la revendication 8.

10. Polymères stabilisés selon la revendication 9, caractérisés en ce que le polymère est un polymère greffé à base d'acrylonitrile, de butadiène et de styrène (ABS) ou un polystyrène à haute résilience.

11. Lubrifiants qui ont été stabilisés au moyen de composés répondant à la formule définie à la revendication 1.